# FASCICULE DE BREVET EUROPEEN

(11) **EP 2 081 564 B1**
(45) Date de publication et mention de la délivrance du brevet: **01.01.2014**
(21) Numéro de dépôt: 07823453.1
(22) Date de dépôt: 29.08.2007
(51) Int. Cl.: A61K 31/198, A61P 3/02

(54) **UTILISATION DE LA CITRULLINE POUR LE TRAITEMENT DES ETATS DE DENUTRITION**
VERWENDUNG VON CITRULLIN ZUR BEHANDLUNG VON UNTERERNÄHRUNGSZUSTÄNDEN
USE OF CITRULLINE FOR TREATING UNDERNUTRITION CONDITIONS

(30) Priorité: 17.10.2006 FR 0609077
(43) Date de publication de la demande: 29.07.2009
(73) Titulaire: Université Paris Descartes, 75270 Paris Cedex 06 (FR); Biocodex, 94250 Gentilly (FR)
(72) Inventeur: MOINARD, Christophe, 92340 Gourg La Reine (FR); JOURDAN, Marion, 75014 Paris (FR); WALRAND, Stéphane, 63970 Aydat (FR); CYNOBER, Luc, 92330 Sceaux (FR)
(74) Mandataire: Grosset-Fournier, Chantal Catherine
(86) Numéro de dépôt international: PCT/FR2007/001407
(87) Numéro de publication internationale: WO 2008/049984

(56) Documents cités:
- EP-A- 1 495 755
- FR-A1- 2 691 359
- FR-M- 6 304
- FR-M- 6 305
- FR-M- 6 306
- US-A- 4 988 724
- US-A- 5 189 025
- US-A1- 2001 056 068
- US-A1- 2004 235 953
- CURIS E ET AL: "Almost all about citrulline in mammals" AMINO ACIDS, SPRINGER VERLAG, AU, vol. 29, no. 3, 8 août 2005 (2005-08-08), pages 177-205, XP002404131 ISSN: 0939-4451
- OSOWSKA S ET AL: "CITRULLINE MODULATES MUSCLE PROTEIN METABOLISM IN OLD MALNOURISHED RATS" AMERICAN JOURNAL OF PHYSIOLOGY: ENDOCRINOLOGY AND METABOLISM, AMERICAN PHYSIOLOGICAL SOCIETY, BETHESDA, MD, US, vol. 291, no. 3, 2006, pages E582-E586, XP008070430 ISSN: 0193-1849
- CYNOBER L ET AL: "Sarcopenie des sujets ages : liberez les acides amines !" NUTRITION CLINIQUE ET METABOLISME, ARNETTE EDITIONS, PARIS, FR, vol. 18, no. 4, décembre 2004 (2004-12), pages 198-204, XP004682355 ISSN: 0985-0562
- CROZIER S J ET AL: "Oral leucine administration stimulates protein synthesis in rat skeletal muscle" JOURNAL OF NUTRITION 2005 UNITED STATES, vol. 135, no. 3, 2005, pages 376-382, XP002427446 ISSN: 0022-3166 cité dans la demande
- GOUTTEBEL M C: "Nutrition artificielle et croissance tumorale" NUTRITION CLINIQUE ET METABOLISME, ARNETTE EDITIONS, PARIS, FR, vol. 11, no. 4, novembre 1997 (1997-11), pages 377-382, XP004884636 ISSN: 0985-0562
- CARDENAS D ET AL: "The mechanism of action of Ornithine [alpha]-ketoglutarate and its role in clinical nutrition" NUTRITION CLINIQUE ET METABOLISME 2002 FRANCE, vol. 16, no. 3, 2002, pages 151-163, XP002427447 ISSN: 0985-0562

## Description

La présente invention a pour objet l'utilisation de L-citrulline pour la préparation d'un médicament destiné au traitement de patients en état de dénutrition liée à une diminution de la synthèse protéique dans le cadre de troubles ou de pathologies ne résultant pas d'une insuffisance intestinale choisis dans le groupe comprenant :
- la malnutrition protéine-énergétique liée à une carence d'apport,
- les cancers, à l'exception du cancer de l'intestin entraînant une insuffisance intestinale,
- la dénervation musculaire,
- les chimiothérapies, à l'exclusion de celles ayant une action au niveau intestinal,
- le diabète,
- l'obésité,
- l'apesanteur,
- les membres immobilisés après fracture,
- la chirurgie réglée, à l'exclusion de la chirurgie digestive intestinale, et
- la dystrophie.

De nombreux états cataboliques sont caractérisés par une dénutrition s'accompagnant d'une diminution de l'anabolisme protéique musculaire. Cette diminution de la protéosynthèse contribue à la fonte musculaire observée dans de nombreux états cataboliques et favorise l'installation d'une cachexie. Or, il est bien connu que cette dernière est un facteur aggravant de morbidité et de mortalité (Schneider SM, Veyres P, Pivot X, Soummer AM, Jambou P, Filippi J et al. Malnutrition is an independent factor associated with nosocomial infections. Br J Nutr 2004; 92:105-111).

Les inventeurs ont précédemment démontré que l'administration par voie entérale de L-citruiline à des rats souffrant d'un état de dénutrition dû à une insuffisance intestinale, permettait à ces rats de reprendre du poids (demande de brevet FR03 08349 publiée sous le no FR 2 857 262).

L'invention décrite dans cette demande de brevet résulte de la mise en évidence du fait que l'administration par voie entérale de L-citrulline à des rats souffrant d'un état de dénutrition dû à une insuffisance intestinale, permettait à ces rats de reprendre du poids.

Les expériences décrites dans la demande FR03 08349 sont effectuées *in vivo* sur des rats ayant subi une résection sévère de l'intestin grêle. Cette résection a pour conséquence une diminution de l'absorption des nutriments et donc une insuffisance intestinale qui conduit à un état de dénutrition.

Les applications de la citrulline découlant de cette démonstration sont celles du traitement :
- du syndrome du grêle court suite à une résection intestinale,
- de la maladie coeliaque,
- des maladies inflammatoires chroniques de l'intestin, telles que la maladie de Crohn, et la rectocolite ulcéreuse,
- de l'insuffisance intestinale liée au vieillissement,
- de l'insuffisance intestinale liée à une irradiation,
à savoir du traitement de pathologies toutes liées à une insuffisance intestinale quelle que soit la cause de cette dernière.

Dans le cadre d'études précédentes chez les rats âgés dénutris, les inventeurs ont plus particulièrement observé une insuffisance intestinale, encore désignée insuffisance intestinale liée au vieillissement, résultant essentiellement d'un mécanisme de séquestration splanchnique des acides aminés qui ne circulent plus en périphérie (Curis E, Nicolis I, Moinard C, Osowska S, Zerrouk N, Benazeth S et al. Almost all about citrulline in mammals. Amino Acids 2005; 29:177-205).

La citrulline n'étant pas captée par l'aire splanchnique, les inventeurs ont émis l'hypothèse que la citrulline pouvait être un vecteur de l'azote en périphérie.

Ils ont ainsi démontré au moyen d'expériences *in vivo* que l'apport de citrulline à des rats âgés dénutris permet de restaurer la synthèse protéique, à savoir que la citrulline permet de retrouver un niveau de synthèse protéique équivalent au niveau basal de synthèse protéique chez le rat âgé sain.

Ces expériences permettaient de suggérer que la citrulline est utilisable dans le cadre du traitement de l'insuffisance intestinale liée au vieillissement (comme mentionné dans la demande de brevet FR03 08349).

Les inventeurs ont étudié dans le cadre de la présente invention l'effet de la citrulline directement sur le muscle par mise en oeuvre d'expériences *in vitro* d'addition de la citrulline sur des muscles isolés de rats adultes sains ou dénutris.

En effet, il n'y a aucune donnée dans la littérature laissant suggérer que le muscle a une quelconque capacité de transformation de la citrulline en une substance active sur la synthèse protéique, ou que la citrulline a une action directe sur la synthèse protéique musculaire.

Les inventeurs ont par conséquent voulu étudier le sort de la citrulline dans le muscle afin d'expliquer le mécanisme métabolique conduisant à un effet sur la stimulation de la synthèse protéique.

C'est ainsi que les Inventeurs ont mis en évidence :
- que le muscle de rats adultes sains ou dénutris n'a pas la capacité de métaboliser la citrulline puisque l'on ne retrouve pas de libération *in vitro* d'acides aminés métaboliquement liés à la citrulline dans le milieu expérimental,
- et que l'addition de citrulline à des muscles de rats adultes dénutris augmente la synthèse protéique de ces muscles jusqu'à 30%, tandis que l'on n'observe aucun effet sur la synthèse protéique par addition *in vitro* de citrulline sur des muscles de rats adultes sains.

Par conséquent, les inventeurs ont pour la première fois démontré que, de façon inattendue, la citrulline n'est pas métabolisée dans le muscle et possède une action directe sur la synthèse protéique musculaire qui est indépendante d'une insuffisance intestinale liée à toute pathologie digestive ou à toute modification du métabolisme digestif.

Ainsi, le but de la présente invention est de fournir un moyen de traiter les états de dénutrition, et plus particulièrement la cachexie lorsque celle-ci est liée à une diminution de synthèse protéique dans le cadre de pathologies non liées à l'insuffisance intestinale.

Un autre but de la description est de fournir un moyen d'augmenter la synthèse protéique intramusculaire anormalement faible chez les patients en état de dénutrition liée à une diminution de la synthèse protéique dans le cadre de pathologies ne résultant pas d'une insuffisance intestinale.

La présente invention concerne l'utilisation de L-citrulline (I) 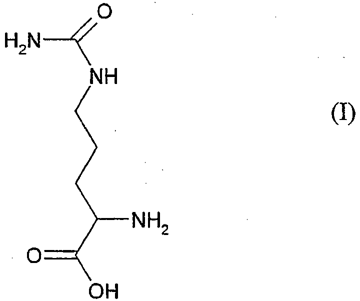 ou un sel pharmaceutiquement acceptable de celle-ci pour la préparation d'un médicament destiné au traitement de patients en état de dénutrition liée à une diminution de la synthèse protéique dans le cadre de pathologies ne résultant pas d'une insuffisance intestinale, choisis dans le groupe comprenant
- malnutrition protéine-énergétique liée à une carence d'apport,
- cancer, à l'exception du cancer de l'intestin entraînant une insuffisance intestinale,
- dénervation musculaire,
- chimiothérapies, à l'exclusion de celles ayant une action au niveau intestinal,
- diabète,
- obésité,
- apesanteur,
- membre immobilisé après fracture,
- chirurgie réglée, à l'exclusion de la chirurgie digestive intestinale, et
- dystrophie.

Au sens de la présente invention on entend par L-citrulline le produit disponible dans le commerce, notamment fourni par Sigma ou le produit naturel issu de végétaux, notamment de la pastèque ou melon d'eau (*Citrullus lanatus*) sous forme notamment de jus, de pulpe ou d'extrait.

On désigne par « insuffisance intestinale » un état pathologique de l'intestin, notamment de l'intestin grêle, dans lequel l'absorption des nutriments est réduite par rapport à la normale, la diminution de l'absorption des nutriments étant liée à une diminution du nombre et/ou de la fonctionnalité de cellules intestinales capables d'assurer cette absorption, cette diminution du nombre et/ou de la fonctionnalité de cellules intestinales étant elle-même due soit à une élimination physique de ces cellules (notamment par chirurgie ou par radiations), soit à un dysfonctionnement pathologique de ces cellules.

La présente description concerne en particulier l'utilisation de L-citrulline pour la préparation d'un médicament destiné à augmenter la synthèse protéique intramusculaire anormalement faible chez les patients en état de dénutrition liée à une diminution de la synthèse protéique dans le cadre de pathologies ne résultant pas d'une insuffisance intestinale.

Les troubles ou pathologies susceptibles d'être traités dans le cadre de la présente invention sont illustrés dans l'article de Couet et al. (Couet C., Attaix D. Le muscle. In: Leverve X, Cosnes J, Erny P, Hasselmann M, editors. Traité de nutrition artificielle de l'adulte. Paris: Mariette Guéna, 1998: 261-274).

La présente invention concerne également une méthode de traitement thérapeutique des troubles et pathologies mentionnées précédemment comprenant l'administration à un patient d'une dose efficace de citrulline ou d'un de ses sels.

La présente invention concerne la L-citrulline pour le traitement des troubles et pathologies mentionnées précédemment.

Selon un autre mode de réalisation, la présente invention concerne l'utilisation susmentionnée de L-citrulline, pour la préparation d'une composition pharmaceutique comprenant, à titre de principe actif, de la L-citrulline, ou un sel pharmaceutiquement acceptable de celle-ci, en association avec un véhicule pharmaceutiquement acceptable.

On désigne notamment par « sel pharmaceutiquement acceptable » des sels de citrulline tels que le malate de citrulline, l'α-cétoglutarate de citrulline, le citrate de citrulline ou l'α-cétoisocaproate de citrulline.

Les véhicules pharmaceutiquement acceptables apparaîtront de manière évidente à l'homme de l'art.

L'invention concerne notamment l'utilisation susmentionnée de L-citrulline, pour la préparation d'une composition pharmaceutique caractérisée en ce que la dose unitaire en L-citrulline est d'environ 2 g à environ 20 g, notamment environ 10 g, pour une posologie d'environ 0,1, g/kg/jour à environ 0,5 g/kg/jour, notamment environ 0,25 g/kg/jour.

L'invention concerne plus particulièrement l'utilisation susmentionnée de L-citrulline, pour la préparation d'une composition pharmaceutique se présentant sous forme sèche ou sous forme de solution aqueuse.

L'invention concerne plus particulièrement l'utilisation susmentionnée de L-citrulline, pour la préparation d'une composition pharmaceutique se présentant sous une forme administrable par voie orale, sous-cutanée, entérale ou parentérale.

L'administration par voie entérale correspond notamment à une administration par sonde naso-gastrique ou naso-intestinale, par gastrotomie ou jéjunostomie, l'administration par voie parentérale correspond notamment à une administration par perfusion intraveineuse centrale, périphérique ou sous-cutanée.

L'invention concerne plus particulièrement l'utilisation susmentionnée de L-citrulline, pour la préparation d'une composition pharmaceutique comprenant également un ou plusieurs autres composés destinés au traitement de la cachexie liée à la dénutrition tels que la leucine, la glutamine, l'arginine, l'ornithine et leurs différents sels utilisables tels que l'α-cétoglutarate ou l'α-cétoisocaproate, seuls ou au sein d'un mélange nutritionnel destiné à la nutrition parentérale, ou d'un mélange destiné à la nutrition entérale ou d'un mélange destiné à la nutrition orale.

Selon un autre mode de réalisation la présente invention concerne une composition pharmaceutique caractérisée en ce qu'elle comprend, à titre de principe actif, de la L-citrulline, ou un sel pharmaceutiquement acceptable de celle-ci, en association avec au moins un autre composé destiné au traitement de la cachexie liée à la dénutrition tels que la leucine, la glutamine, l'arginine, l'ornithine et leurs différents sels utilisables tels que l'α-cétoglutarate ou l'α-cétoisocaproate, seuls ou au sein d'un mélange nutritionnel destiné à la nutrition parentérale, ou d'un mélange destiné à la nutrition entérale ou d'un mélange destiné à la nutrition orale, et avec un véhicule pharmaceutiquement acceptable.

Selon un autre mode de réalisation l'invention concerne des produits comprenant :
- de la L-citrulline ou un sel pharmaceutiquement acceptable de celle-ci,
- et au moins un autre composé destiné au traitement de la cachexie liée à la dénutrition tels que la leucine, la glutamine, l'arginine, l'ornithine et leurs différents sels utilisables tels que l'α-cétoglutarate ou l'α-cétoisocaproate, seuls ou au sein d'un mélange nutritionnel destiné à la nutrition parentérale, ou d'un mélange destiné à la nutrition entérale ou d'un mélange destiné à la nutrition orale, comme produits de combinaison pour une utilisation simultanée, séparée, ou étalée dans le temps, dans le cadre du traitement de l'insuffisance intestinale.

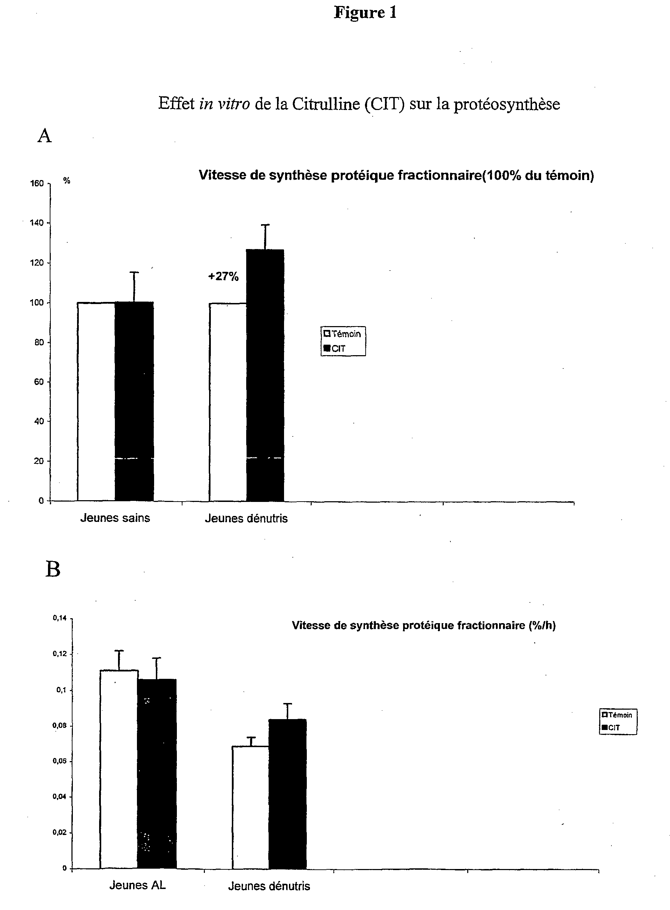

L'invention est illustrée à l'aide des exemples 1 et 2 et des figures 1 à 3 qui suivent.

### Description des figures

La figure 1A représente la synthèse protéique fractionnaire (FSR) d'*epitrochlearis* issus de rats sains (colonnes de gauche) ou dénutris (colonnes de droite) et incubés en présence de citrulline (colonnes noires) ou en absence de citrulline (témoin - colonnes blanches) mesurée selon le mode opératoire de l'exemple 1. Les résultats sont exprimés en %/heure.
La figure 1B représente la synthèse protéique fractionnaire (FSR) d'*epitrochlearis* issus de rats sains (colonnes de gauche) ou dénutris (colonnes de droite) et incubés en présence de citrulline (colonnes noires) ou en absence de citrulline (témoin - colonnes blanches) mesurée selon le mode opératoire de l'exemple 1. Les résultats sont exprimés en %/témoin.

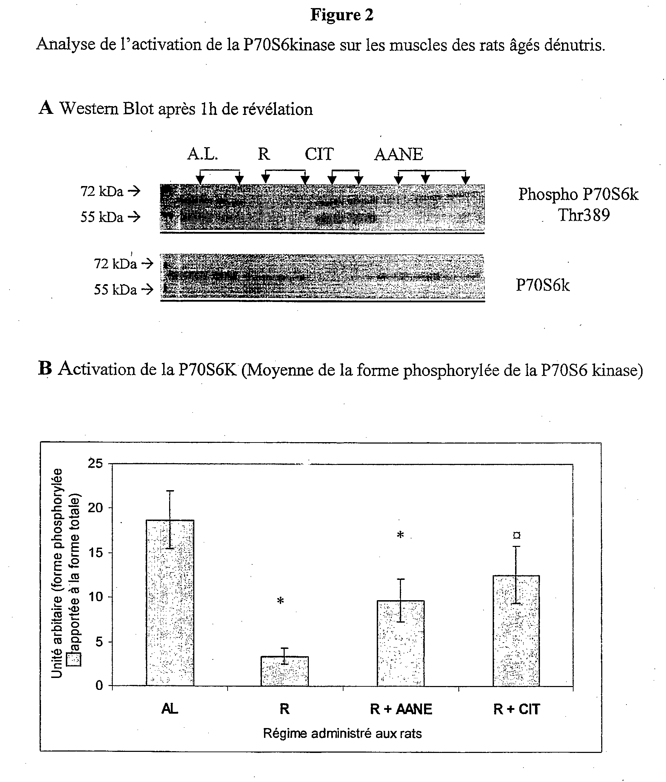

La figure 2A représente le Western Blot après 1h de révélation illustrant l'activation de la P70S6kinase sur les muscles des rats âgés dénutris mesurée selon le mode opératoire décrit dans l'exemple 2.
La figure 2B est la représentation graphique de l'activation de la P70S6kinase sur les muscles des rats âgés dénutris mesurée selon le mode opératoire décrit dans l'exemple 2. AL : groupe témoin ; R : groupe soumis à une restriction alimentaire ; R + AANE : groupe soumis à une restriction alimentaire puis à un régime standard enrichi en acides aminés non essentiels ; R + CITR : groupe soumis à une restriction alimentaire puis à un régime standard enrichi en citrulline (5g/kg/j). *Tests statistiques*: ANOVA + PLSD test de Fisher: * *versus* AL, p<0,05 ; ¤ *versus* R, p<0,05

### Exemple 1 Régulation de la protéosynthèse musculaire par la citrulline mesurée in vitro sur muscle isolé périfusé.

### 1.1. Matériel et méthodes

Traitement des animaux : Des rats mâles Sprague-Dawley (Charles River Laboratoires, L'Arbresles, France) âgés de 3 mois (n=20) sont placés dans des cages individuelles, en ambiance thermostatée (23° ± 1°C), et soumis à un cycle lumière/obscurité de 12h (obscurité de 8h à 20h).

L'acclimatation des rats est effectuée pendant 2 semaines, au cours desquelles la consommation alimentaire spontanée est mesurée. Ils sont nourris avec un régime standard (A04, UAR, Villemoisson-sur-Orge, France) contenant 17% de protéines, 3% de lipides, 59% de glucides et 21% d'eau, fibres, vitamines et minéraux. La prise alimentaire moyenne, durant cette période est de 28 g/jour pour les rats adultes.

A la fin de la période d'acclimatation, les rats sont randomisés en 2 groupes : un groupe contrôle formés de rats nourris *ad libitum* (AL) et un groupe soumis à une restriction alimentaire durant la même période : les rats sont nourris à 50% des ingesta spontanés pendant 6 semaines avec un régime à 5% de protéines (Walrand S, Chambon-Savanovitch C, Felgines C, Chassagne J, Raul F, Normand B et al. Aging: a barrier to renutrition? Nutritional and immunologic evidence in rats. Am J Clin Nutr 2000; 72:816-824).

Muscles isolés incubés. Les muscles ont été incubés selon une méthode préalablement utilisée (Minet-Quinard R, Moinard C, Villie F, Vasson MP, Cynober L. Metabolic pathways implicated in the kinetic impairment of muscle glutamine homeostasis in adult and old glucocorticoid-treated rats. Am J Physiol Endocrinol Metab 2004; 287:E671-E676). L'*epitrochlearis* est utilisé car il est le plus adapté à ce type d'étude. Après dissection, les *epitrochlearis* sont incubés dans 3 mL de tampon Krebs-Ringer (119 mM NaCl ; 4,8 mM KCl ; 1,25 mM MgSO₄ ; 25 mM NaHCO₃ ; 1,24 mM NaHPO₄ ; 1,0 mM CaCl₂ ; 2 mM HEPES, pH 7,4), contenant également du glucose (8 mM), de l'insuline (0,01 U/ml) et de l'albumine bovine sérique (BSA) (0,1% p/v). Les muscles sont pré-incubés pendant 30 minutes à 37°C avec 95%O₂:5%CO₂. Les muscles sont ensuite transférés dans un tube contenant 3 mL de milieu d'incubation (contenant de la ¹³C-phénylalanine (1 mM) avec ou sans 2,5 mM de citrulline) et sont incubés pendant 2 heures. Au terme de l'incubation, les muscles sont recueillis et conservés à -80°C jusqu'à la mesure de l'incorporation de la ¹³C-phénylalanine par spectrométrie de masse pour déterminer la synthèse protéique fractionnaire (FSR) (Guillet C, Boirie Y, Walrand S. An integrative approach to in-vivo protein synthesis measurement: from whole tissue to specific proteins. Curr Opin Clin Nutr Metab Care 2004; 7:531-538). Par ailleurs, les acides aminés sont dosés dans le milieu d'incubation par chromatographie échangeuse d'ions.

### 1.2. Résultats

Ils sont donnés dans la figure 1.

La citrulline n'est pas métabolisée par le muscle car aucun acide aminé métaboliquement lié à la citrulline n'est libéré dans le milieu d'incubation (résultats non montrés).

Les résultats montrent que, conformément aux données de la littérature, la dénutrition diminue la synthèse protéique musculaire chez les rats adultes (colonne blanche jeune sain à comparer à colonne blanche jeunes dénutris figure 1A).

Les résultats des expériences susmentionnées montrent que l'administration de L-citrulline permet d'augmenter la protéosynthèse musculaire chez des rats dénutris présentant une diminution de leur protéosynthèse musculaire (+27% figure 1A comparaison colonne blanche et colonne noire de jeunes dénutris). Ce travail, ayant été réalisé *ex vivo* sur des muscles isolés incubés, permet de montrer que la L-citrulline a une action directe au niveau musculaire. Ce résultat est inattendu et totalement imprévisible au vue des données de la littérature, car seule la leucine (acide aminé essentiel) possède une telle propriété (Crozier SJ, Kimball SR, Emmert SW, Anthony JC, Jefferson LS. Oral leucine administration stimulates protein synthesis in rat skeletal muscle. J Nutr 2005; 135:376-382). Or, la L-citrulline est un acide aminé dont la structure est très différente de la leucine ; elle n'entre pas dans la composition des protéines, et est quasiment absente de l'alimentation.

### Exemple 2 : Etude in vivo démontrant une action directe sur la synthèse protéique via l'activation de la voie inTOR

### 2.1. Matériel et méthodes

Traitement des animaux : Quarante rats Sprague-Dawley mâles âgés de 19 mois (Charles River, L'Arbresle, France) ont été utilisés. Durant une période d'acclimatation de 2 semaines, les animaux ont été nourris *ad libitum* avec un régime standard (UARA04, Usine d'Alimentation Rationnelle, Villemoisson-sur-Orge). La mesure des ingesta spontanés a été effectuée régulièrement. Suite à cette période, 30 rats sont soumis à une restriction alimentaire de 50% pendant 12 semaines. Le sacrifice de 10 rats, servant de groupe témoin (groupe R), a été réalisé à la fin de la période de restriction alimentaire. Les 20 autres animaux ont été renourris pendant une semaine avant d'être sacrifiés. Les régimes isoazotés et isocaloriques étaient soit une nutrition standard enrichie en citrulline 5g/kg/j (n=10, groupe R+CIT), soit une nutrition standard enrichie en acides aminés non essentiels (Ala, Gly, His, Asp, Ser dans un ratio équimolaire) (n=10, groupe R+AANE). Dix rats constituent le groupe témoin (A.L) ; ils sont nourris *ad libitum* tout au long de l'étude avant d'être sacrifiés. Les rats sont ensuite sacrifiés. Les muscles *tibialis* sont prélevés et congelés rapidement dans l'azote liquide, puis stockés à -80°C jusqu'à leur analyse.

Extraction des protéines et dosage : Les muscles sont broyés en azote liquide à l'aide d'un mortier. La poudre ainsi obtenue est pesée, puis reprise dans 10 volumes de tampon de solubilisation contenant un cocktail d'inhibiteurs de phosphatases et de protéases. Les échantillons sont placés au minimum 1 heure sur une roue en chambre froide. Après une centrifugation de 30 minutes à +4°C à 13000 g, le surnageant est réparti en fractions aliquotes puis conservé à -80°C.

Les protéines sont dosées par la méthode de l'acide bicinchoninique.

Une fraction aliquote de chaque échantillon est dénaturée au bain-marie à 100°C pendant 5 minutes dans un tampon de dénaturation contenant du β-mercaptoéthanol (5%) et du tampon Laemmli (Laemmli Sample Buffer).

L'analyse de l'activation de la voie mTOR (mammalian Target of Rapamycin) est réalisée par Western Blot mettant en évidence les formes phosphorylées des différentes cibles de mTOR. L'intégrité des protéines est vérifiée par électrophorèse en gel de polyacrylamide (SDS-PAGE), suivie d'une coloration au Bleu de Coomassie.

Technique de Western Blot : 20 µg de protéines dénaturées sont déposés sur un gel de polyacrylamide 10% et 12 % respectivement pour l'étude de la p70^{S6K} et de la rpS6 (formes phosphorylée ou totale).
- *Migration des protéines par électrophorèse :* La migration est réalisée à 20mA pendant 2 heures dans un tampon de migration 1X (Tris 25mM, Glycine 192mM, SDS 0,1%).
- *Transfert des protéines sur membrane de nitrocellulose :* Les gels sont alors transférés sur membrane de nitrocellulose. Pour cela, ils sont chacun placés en cassette. Le transfert s'effectue à froid dans du tampon de transfert 1X (Tris 25mM, Glycine 192mM, SDS 0,01%, éthanol absolu 20%), à 120mA pendant minimum 1h30. La qualité du transfert est visualisée par une coloration au rouge ponceau.
- *Immunodétection* : Les membranes sont pré incubées une heure dans un tampon approprié [Tampon Tris (Tris Buffer Saline Tween 1X (TBST)] : Tris 1mM, NaCl 15mM, Tween20 0,5%, pH8 ; 1% lait écrémé en poudre, 4%BSA) afin de saturer les sites non spécifiques. Elles sont ensuite incubées une nuit à 4°C dans un tampon d'hybridation dépendant de la forme protéique à étudier. Pour l'étude de la forme phosphorylée, le tampon TBST 1X, 1% lait écrémé, 4% BSA, est mélangé à l'anticorps primaire. Pour l'étude de la forme totale, le tampon TBSA (TBST 1X, 5% lait écrémé) sert au mélange avec l'anticorps primaire (P70 S6kinase total #9202 dilué au 1/125^{e} ; anti Phospho-P70 S6kinase (Thr389) #9234 dilué au 1/250^{e} ; rpS6 (5G10) total #2217 dilué au 1/2000^{e} ; Phospho-rpS6 (Ser240/244) #2215 dilué au 1/2000^{e}). Après plusieurs rinçages dans une solution de TBST 1X, 3 lavages de 20 minutes sont effectués dans cette même solution. Les membranes sont ensuite incubées ¾ d'heure avec le second anticorps couplé à la peroxydase. De nouveau, elles sont rincées 2 fois dans du TBST 1X, puis lavées 3 fois minimum 20 minutes dans cette même solution. La révélation sur film radiographique se réalise en chambre noire à l'aide du kit ECL.

### 2.2. Résultats

Ils sont donnés dans la figure 2.

Les résultats montrent que la restriction alimentaire (groupe R) diminue de manière significative l'activité de la P70S6 kinase (- 83 % par rapport aux témoins du groupe A.L.).

Un régime alimentaire supplémenté en acides aminés non essentiels (groupe R + AANE) restaure une partie de cette activité mais de manière non significative, ladite activité restant significativement inférieure à celle observée chez les témoins (-45 % par rapport au groupe témoins A.L.).

Au contraire un régime alimentaire supplémenté en citrulline à la dose de 5g/kg/j (groupe R + CIT) restaure de manière significative cette activité (+ 417 % par rapport au groupe R), ladite activité n'étant pas significativement différente de celle observée chez les témoins (groupe A.L.).

Ainsi ces résultats montrent que la citrulline a une activité directe sur la synthèse protéique via l'activation du système mTOR.

## Revendications

1. Utilisation de L-citrulline (I) 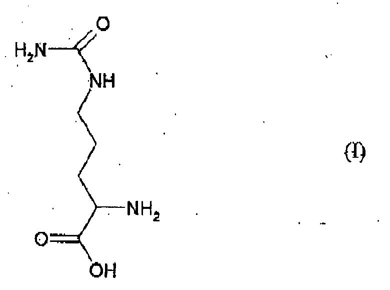 ou d'un sel pharmaceutiquement acceptable de celle-ci pour la préparation d'un médicament destiné au traitement de patients en état de dénutrition liée à une diminution de la synthèse protéique dans le cadre de troubles ou de pathologies ne résultant pas d'une insuffisance intestinale choisis dans le groupe comprenant :
- la malnutrition protéine-énergétique liée à une carence d'apport,
- les cancers, à l'exception du cancer de l'intestin entraînant une insuffisance intestinale,
- la dénervation musculaire,
- les chimiothérapies, à l'exclusion de celles ayant une action au niveau intestinal.
- le diabète,
- l'obésité,
- l'apesanteur,
- les membres immobilisés après fracture,
- la chirurgie réglée, à l'exclusion de la chirurgie digestive intestinale, et
- la dystrophie.

2. Utilisation de L-citruline selon la revendication 1 **caractérisée en ce que** la composition pharmaceutique comprend à titre, de principe actif, de la L-citrulline, ou un sel pharmaceutiquement acceptable de celle-ci, en association avec un véhicule pharmaceutiquement acceptable.

3. Utilisation de L-citrulline selon l'une des revendications 1 à 2, **caractérisée en ce que** la dose unitaire en L-citrulline est d'environ 2 g à environ 20 g, pour une posologie d'environ 0,1 g/kg/jour à environ 0,5 g/kg/Jour.

4. Utilisation de L-citrulline selon la revendication 3 **caractérisée en ce que** la dose unitaire en L-citrulline est d'environ 10 g, pour une posologie d'environ 0,1 g/kg/jour à environ 0,5 g/kg/jour.

5. Utilisation de L-citrulline selon la revendication 3 **caractérisée en ce que** la dose unitaire en L-citrulline est d'environ 2 g à environ 20 g pour une posologie d'environ 0,25 g/kg/jour.

6. Utilisation de L-citrulline selon la revendication 3 **caractérisée en ce que** la dose unitaire en L-citrulline est d'environ 10 g, pour une posologie d'environ 0,25 g/kg/jour.

7. Utilisation de L-citrulline selon l'une des revendications 1 à 6, **caractérisée en ce que** la composition pharmaceutique se présente sous forme sèche ou sous forme de solution aqueuse.

8. Utilisation de L-eitrulline selon l'une des revendications 1 à 6, **caractérisée en ce que** la composition pharmaceutique se présente sous une forme administrable par voie orale, sous-cutanée, entérale ou parentérale.

9. Utilisation de L-citrulline selon l'une des revendications 1 à 8, **caractérisée en ce que** la composition pharmaceutique comprend également un ou plusieurs autres composés destinés au traitement de la cachexie liée à la dénutrition tels que la leucine, la glutamine, l'arginine, l'ornithine et leurs différents sels utilisables tels que l'α-cétoglutarate ou l'α-cétoisocaproate, seuls ou au sein d'un mélange nutritionnel destiné à la nutrition parentérale, ou d'un mélange destiné à la nutrition entérale ou d'un mélange destiné à la nutrition orale.

10. L-citrulline pour son utilisation dans le traitement de patients en état de dénutrition liée à une diminution de la synthèse protéique dans le cadre de troubles ou de pathologies ne résultant pas d'une insuffisance intestinale choisis dans le groupe comprenant :
- la malnutrition protéino-énergétique liée à une carence d'apport,
- les cancers, à l'exception du cancer de l'intestin entraînant une insuffisance intestinale,
- la dénervation musculaire,
- les chimiothérapies, à l'exclusion de celles ayant une action au niveau intestinal,
- le diabète,
- l'obésité,
- l'apesanteur,
- les membres immobilisés après fracture,
- la chirurgie réglée, à l'exclusion de la chirurgie digestive intestinale, et
- la dystrophie.

## Patentansprüche

1. Verwendung von L-Citrullin (I) 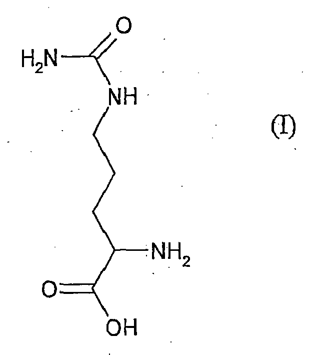 oder eines pharmazeutisch akzeptablen Salzes davon zur - Herstellung eines Medikaments, das zur Behandlung von Patienten im Unterernährungszustand bestimmt ist, der mit einer Verringerung der Proteinsynthese im Rahmen von Störungen oder Pathologien, die nicht aus einer Darminsuffizienz resultieren, verbunden ist, ausgewählt aus der Gruppe, umfassend
- protein- und energiebezogene Fehlernährung, die mit einer unzureichenden Zufuhr verbunden ist,
- Krebsformen, mit Ausnahme von Darmkrebs, die zu einer Darminsuffizienz führen,
- Muskeldenervierung,
- Chemotherapien, mit Ausnahme derer, die auf den Darm einwirken,
- Diabetes,
- Fettleibigkeit,
- Schwerelosigkeit,
- nach einem Bruch ruhig gestellte Gliedmaßen,
- elektive Chirurgie, mit Ausnahme der gastrointestinalen Chirurgie und
- Dystrophie.

2. Verwendung von L-Citrullin nach Anspruch 1, **dadurch gekennzeichnet, dass** die pharmazeutische Zusammensetzung als Wirkstoff L-Citrullin oder ein pharmazeutisch akzeptables Salz davon in Verbindung mit einem pharmazeutisch akzeptablen Vehikel umfasst.

3. Verwendung von L-Citrullin nach einem der Ansprüche 1 bis 2, **dadurch gekennzeichnet, dass** die L-Citrullin-Einheitsdosis für eine Posologie von etwa 0,1 g/kg/Tag bis etwa 0,5 g/kg/Tag bei etwa 2 g bis etwa 20 g liegt.

4. Verwendung von L-Citrullin nach Anspruch 3, **dadurch gekennzeichnet, dass** die L-Citrullin-Einheitsdosis für eine Posologie von etwa 0,1 g/kg/Tag bis etwa 0,5 g/kg/Tag bei etwa 10 g liegt.

5. Verwendung von L-Citrullin nach Anspruch 3, **dadurch gekennzeichnet, dass** die L-Citrullin-Einheitsdosis für eine Posologie von etwa 0,25 g/kg/Tag bei etwa 2 g bis etwa 20 g liegt.

6. Verwendung von L-Citrullin nach Anspruch 3, **dadurch gekennzeichnet dass** die L-Citrullin-Einheitsdosis für eine Posologie von etwa 0,25 g/kg/Tag bei etwa 10 g liegt.

7. Verwendung von L-Citrullin nach einem der Ansprüche 1 bis 6, **dadurch gekennzeichnet, dass** die pharmazeutische Zusammensetzung eine trockene Form oder die Form einer wässrigen Lösung aufweist.

8. Verwendung von L-Citrullin nach einem der Ansprüche 1 bis 6, **dadurch gekennzeichnet, dass** die pharmazeutische Zusammensetzung eine Form aufweist, die oral, subkutan, enteral oder parenteral verabreicht werden kann.

9. Verwendung von L-Citrullin nach einem der Ansprüche 1 bis 8, **dadurch gekennzeichnet, dass** die pharmazeutische Zusammensetzung auch eine oder mehrere andere Verbindungen umfasst, die zur Behandlung von mit Mangelernährung verbundener Kachexie bestimmt sind, wie etwa Leucin, Glutamin, Arginin, Ornithin und deren verschiedenen verwendbaren Salze, wie etwa α-Ketoglutarat oder α-Ketoisocaproat, allein oder in einem Nahrungsgemisch, das zur parenteralen Ernährung bestimmt ist, oder einem Gemisch, das zur enteralen Ernährung bestimmt ist, oder einem Gemisch, das zur oralen Ernährung bestimmt ist.

10. L-Citrullin zur Verwendung bei der Behandlung von Patienten im Unterernährungszustand, der mit einer Verringerung der Proteinsynthese verbunden ist, im Rahmen von Störungen oder Pathologien, die nicht aus einer Darminsuffizienz resultieren, ausgewählt aus der Gruppe, umfassend:
- protein- und energiebezogene Fehlernährung, die mit einer unzureichenden Zufuhr verbunden ist,
- Krebsformen, mit Ausnahme von Darmkrebs, die zu einer Darminsuffizienz führen,
- Muskeldenervierung,
- Chemotherapien, mit Ausnahme derer, die auf den Darm einwirken,
- Diabetes,
- Fettleibigkeit,
- Schwerelosigkeit,
- nach einem Bruch ruhig gestellte Gliedmaßen,
- elektive Chirurgie, mit Ausnahme der gastrointestinalen Chirurgie und
- Dystrophie.

## Claims

1. Use of L-citrulline (I) 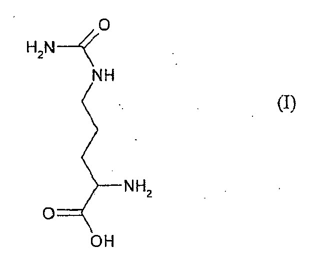 or of a pharmaceutically acceptable salt thereof in the preparation of a drug intended for the treatment of patients in a state of undernutrition as linked to a lowering of the protein synthesis within the framework of disorders or pathologies which do not result from an intestinal insufficiency selected from the group comprising:
- protein energy malnutrition as linked to an intake deficiency,
- cancers, except intestinal cancer leading to an intestinal insufficiency,
- muscle denervation,
- chemotherapies, except those which have an action at the intestinal level,
- diabetes,
- obesity,
- weightlessness,
- limbs which are immobilized after a fracture,
- regulated surgery, except intestinal digestive surgery, and
- dystrophy.

2. Use of L-citrulline according to claim 1, **characterized in that** the pharmaceutical composition comprises, as an active ingredient, L-citrulline or a pharmaceutically acceptable salt thereof, in association with a pharmaceutically acceptable excipient.

3. Use of L-citrulline according to any of claims 1 or 2, **characterized in that** the unit dose of L-citrulline is between ca. 2 g to ca. 20 g, for a dosage regimen of ca. 0.1 g/kg/day to ca. 0.5 g/kg/day.

4. Use of L-citrulline according to claim 3, **characterized in that** the unit dose of L-citrulline is ca. 10 g, for a dosage regimen of ca. 0.1 g/kg/day to ca. 0.5 g/kg/day.

5. Use of L-citrulline according to claim 3, **characterized.in that** the unit dose of L-citrulline is ca. 2 g to ca. 20 g, for a dosage regimen of ca. 0.25 g/kg/day.

6. Use of L-citrulline according to claim 3, **characterized in that** the unit dose of L-citrulline is ca. 10 g, for a dosage regimen of ca. 0.25 g/kg/day.

7. Use of L-citrulline according to any of claims 1 - 6, **characterized in that** the pharmaceutical composition is obtained in the form of a dry composition or of an aqueous solution.

8. Use of L-citrulline according to any of claims 1 - 6, **characterized in that** the pharmaceutical composition may be found in a form which may be administered orally, subcutaneously, enterally or parenterally.

9. Use of L-citrulline according to any of claims 1 - 8, **characterized in that** the pharmaceutical composition also comprises one or several other compounds intended for the treatment of cachexia as linked to undernutrition, such as leucine, glutamine, arginine, ornithine and their various acceptable salts such as α-ketoglutarate or a α-ketoisocaproate, whether isolated or within a nutritional mixture intended for parenteral nutrition, or a mixture intended for enteral nutrition, or a mixture intended for oral nutrition.

10. L-citrulline for its use in treating patients in state of undernutrition as linked to a lowering of the protein synthesis within the framework of disorders or pathologies which do not result from an intestinal insufficiency selected from the group comprising:
- protein energy malnutrition as linked to an intake deficiency,
- cancers, except intestinal cancer leading to an intestinal insufficiency,
- muscle denervation,
- chemotherapies, except those which have an action at the intestinal level,
- diabetes,
- obesity,
- weightlessness,
- limbs which are immobilized after a fracture,
- regulated surgery, except intestinal digestive surgery, and
- dystrophy.
